# EUROPEAN PATENT SPECIFICATION

(11) **EP 4 128 098 B1**
(45) Date of publication and mention of the grant of the patent: **18.02.2026**
(21) Application number: 21717320.2
(22) Date of filing: 26.03.2021
(51) Int. Cl.: G06Q 10/06, G06Q 50/02, A01K 1/00, G16H 10/00, A01K 29/00

(54) **METHOD AND QUALITY SYSTEM FOR DETERMINING A QUALITY PARAMETER OF AN AGRICULTURAL GROUP**
VERFAHREN UND QUALITÄTSSYSTEM ZUM ERMITTELN EINES QUALITÄTSPARAMETERS EINER LANDWIRTSCHAFTLICHEN GRUPPE
PROCÉDÉ ET SYSTÈME DE QUALITÉ POUR DÉTERMINER UN PARAMÈTRE DE QUALITÉ DE GROUPE AGRICOLE

(30) Priority: 31.03.2020 DK PA202070199
(43) Date of publication of application: 08.02.2023
(73) Proprietor: Force Technology, 2970 Hørsholm (DK)
(72) Inventor: SKOV, Julia, 2970 Hørsholm (DK); ANDERSEN, Karsten Brandt, 2970 Hørsholm (DK)
(74) Representative: Patrade A/S
(86) International application number: PCT/DK2021/050090
(87) International publication number: WO 2021/197557

(56) References cited:
- WO-A1-2015/171834

## Description

### TECHNICAL FIELD

The invention relates to a system and a method for determining a quality parameter of a flock of animals.

### BACKGROUND ART

In the veterinary industry, it is standard to acquire samples from selected animals from a flock of animal to determine if there are any infections in the flock. The farmer may for example take samples of blood or feces of an animal that appears to be sick to determine if the animal (and hence the flock) is infected and optionally to determine which treatment may best suited to handle the situation. However, often early signs of an onset infection are overlooked - especially when the farmer is not fully aware what to look for. Diseases may develop sub clinically for some time before symptoms are observable by the farmer.

Vidic et al. "Advanced biosensors for detection" of pathogens related to livestock and poultry. Vet Res (2017) 48:11 DOI 10.1186/s13567-017-0418-5, presents a review of developed biosensors for detection of infection of pathogenic microorganisms of wildlife, livestock, and human populations. The article is focused on biosensors that may be applied for domestic animal pathogen diagnostics and discloses different types of sensors, such as a colorimetric sensor for detection of influenza A virus, Lateral flow strips for detection of influenza A and B viruses as well as sensors applying PCR amplification for nucleic acid amplification and virus detection.

Olsen et al. "Detection of Campylobacter Bacteria in Air Samples for Continuous Real-Time Monitoring of Campylobacter Colonization in Broiler Flocks APPLIED AND ENVIRONMENTAL MICROBIOLOGY, Apr. 2009, p. 2074-2078 Vol. 75, No. 7. doi:10.1128/AEM.02182-08 describes an assay of detection of Campylobacter in poultry houses. The detections ware based on PCR amplification of nucleic acids from air borne particles collected from the poultry houses.

US 2010/0198023 describes a health monitoring system comprising a plurality of different sensors mounted at scattered points throughout the livestock site. Scattering points are chosen and marked on a 3D map of the site, prepared prior to the system's positioning. The sensors are monitoring physical behaviors and activities of the animals using data collecting unit types selected from the group consisting of acoustic sensors, vitality meters, ammonia sensors, visual sensors and scent sensors. This system needs to be pre-installed and further requires a high number of sensors. In addition, it requires measurement of individual animals to operate effectively.

WO2015171834 discloses a method according to the preamble of clam 1.

### DISCLOSURE OF INVENTION

An objective of the present invention is to provide an effective system and method for optimizing production of animal goods, such as meat, dairy, eggs, wool, and leather.

In an embodiment, it is an objective to provide system suitable for use in optimization of production of animal goods, which system is relatively inexpensive and simple to use for a farmer.

In an embodiment, it is an objective to provide system suitable for use in optimization of production of animal goods, which system is reliable.

In an embodiment, it is an objective to provide method for use in optimization of production of animal goods, which method is relatively simple to perform and provides reliable results.

These and other objects have been solved by the inventions or embodiments thereof as defined in the claims and as described herein below.

It has been found that the inventions or embodiments thereof have a number of additional advantages, which will be clear to the skilled person from the following description.

It should be emphasized that the term "comprises/comprising" when used herein is to be interpreted as an open term, i.e. it should be taken to specify the presence of specifically stated feature(s), such as element(s), unit(s), integer(s), step(s) component(s) and combination(s) thereof, but does not preclude the presence or addition of one or more other stated features.

Reference made to "some embodiments" or "an embodiment" means that a particular feature, structure, or characteristic described in connection with such embodiment(s) is included in at least one embodiment of the subject matter disclosed. Thus, the appearance of the phrases "in some embodiments" or "in an embodiment" in various places throughout the specification is not necessarily referring to the same embodiment(s). Further, the skilled person will understand that particular features, structures, or characteristics may be combined in any suitable manner within the scope of the invention as defined by the claims.

The term "substantially" should herein be taken to mean that ordinary product variances and tolerances are comprised.

The terms "test" and "assay" are used interchangeable.

The phrase "as a function of time" means over a time range, preferably a time range comprising several determinations, preferably over at least 1 hour, preferably over at least 1 day and preferably longer.

Particles or airborne particles means herein droplet of liquid and/or solids, such as aerosols. The airborne particle includes "heterodisperse" aerosols as well as "polydisperse aerosols of mixed chemical constituents. The particles may be aggregates or clusters. The particles may be visible or invisible to the naked eye and may have any size provided that they are capable of being airborne for a sufficient time for being collected. A particle may be a bacteria in moist or dry state. Advantageously, the airborne particles has a settling time of at least about 10 seconds for settling 1 m in still air. Throughout the description or claims, the singular encompasses the plural unless otherwise specified or required by the context.

**All** features of the invention and embodiments of the invention as described herein, including ranges and preferred ranges, may be combined in various ways within the scope of the invention, unless there are specific reasons not to combine such features.

**The** invention comprises a quality system for determining a quality parameter of agricultural group. **The** quality system comprising a computer system in data communication with an analyzing system adapted for analyzing samples of particles collected from air from an agricultural group location comprising the agricultural group,

**The** analyzing system is configured
- for receiving a plurality of samples, each sample comprises particles or fragment(s) of particles collected from air from the agricultural group location at plurality of consecutive selected time slots and each sample being correlated with a time attribute representing a time of collection;
- for performing at least one quantitative, biological element determination of each of the received samples; and
- for transmitting sub-sets of data, each comprising data representing the at least one quantitative, biological element determination of a sample and the time attribute of the sample to the computer system;

**The** computer system is configured
- for receiving the sub-sets of data,;
- for correlating the received sub-sets of data with at least one set of reference data representing; and
- for determining the quality parameter of the agricultural group,

**The** sets of reference data represents reference quantity of the biological element as a function of time correlated to the quality parameter comprising at least one threshold quality parameter of the at least one biological element as a function of time and wherein the determination of the quality parameter comprises determining quantity of the at least one biological element as a function of time and wherein the computer further is configured for determine the quality parameter relative to the at least one threshold quality parameter.

The threshold quality parameter is in the following also referred to simply as the "threshold".

As it will be explained further below, the agricultural group is primarily selected from a flock of animals, a feed lot and/or an animal bedding lot.

The inventors of the present invention have found that particles in the environment near the agricultural group e.g. near the flock of animals, near the feed lot and/or near the bedding lot, comprises a highly representative biological representation, which if collected and analyzed as described herein may provide information of one or more quality parameters of the entire flock of animals, without requirements for performing measurements of individual animals.

Advantageously, the analyzing system or at least a part of the analyzing system form part of the quality system.

In an aspect the quality system is a quality system adapted for determining a quality parameter of a feed lot.

The term "feed lot" is herein used to designate a volume and/or mass of feed for animals, such as a batch of feed. Usually a lot of feed will be a relatively large amount of feed such that an amount that are sufficient for more than one day for a flock of animals. Examples of feed lots are provided below.

In an aspect the quality system is a quality system adapted for determining a quality parameter of an animal bedding lot.

The term "bedding lot" or "animal bedding lot" is herein used to designate a volume and/or mass of bedding for animals, such as a batch of bedding material. Examples of animal bedding advantageously comprises straw, sawdust, hemp straw, fleece, sand, wood shaving, peanut hulls, shredded sugar cane, peat moss or mixtures comprising any of these.

It has been found that by analyzing particles collected from air from the feed lot location at consecutive time for the quantity of least one biological element and correlating the received consecutively sub-sets of the data with a set of reference data representing quantity of the biological element as a function of time correlated to the quality parameter(s) in question a very accurate determination of the quality parameter(s) may be found without performing any destructive or damaging analysis on any parts of the feed lot. The development of the quantity of various biological elements has been found to be very characteristic for quality parameters of feed lots. It has further been found that even small signs of decrease in the quality parameter may be determined at a very early state, which may make it possible to take steps to reduce risk of a reduction in quality. The quality system may thereby provide an instrument for maintaining or even improving quality of one or more quality parameters of feed lots. If for example a feed lot has a sign of decrease in quality parameter discovered by the quantitative determinations of at least one biological element as a function of time, it may for example be decided to avoid far distance transportation of the feed lot and instead use the feed lot shortly, thereby saving the feed of the feed lot to be wasted and/ or saving a vain transport. Similar benefits may be obtained where the agricultural group is an animal bedding lot

In addition the risk of serving feed of undesired quality for animals may be reduced or even fully avoided.

The system is advantageously configured for acquiring discrete particle samples as a function of time (i.e. at selected time slots) and obtaining quantitative determinations of biological element(s) of the sample acquired over time at selected time slots. Thereby quantity of biological element(s) may be determined as a function of time represented by the sub sets of data.

In an embodiment, where the quality system shows a decrease in quality parameter, the person responsible for the feed lot and/or the animal bedding lot, e.g. the feed/animal bedding manufacturer, feed/animal bedding transporter or the farmer may safe the feed lot and/or animal bedding lot by certain action, such as subjecting the lot to a heat treatment to maintain best possible quality.

Advantageously, the analyzing system or at least a part of the analyzing system form part of the quality system.

The quality system adapted for determining a quality parameter of an agricultural group may advantageously be a combined quality system. When referring to the quality system in the following the quality system referred to includes any of the quality system adapted for determining a quality parameter of a flock of animals, the quality system adapted for determining a quality parameter of a feed lot, the quality system adapted for determining a quality parameter of an animal bedding lot and/or the combined quality system unless otherwise clear from the context.

The threshold quality parameter comprises advantageously at least one threshold quantity of the at least one biological element and/or a threshold drift of quantity of the at least one biological element as a function of time.

In an embodiment, the threshold quality parameter comprises at least one threshold change of quantity of the biological element as a function of time, optionally correlated to a selected level of the quality parameter.

Advantageously, the computer system and/or a data cloud storage in data communication with the computer system stores the at least one set of reference data. Preferably the sets of reference data each comprises reference data representing quantity of the biological element as a function of time correlated to the quality parameter.

The computer system and/or data cloud storage may store at least one set of reference data, correlated to at least one type of agricultural group and/or type of agricultural group location.

In an embodiment, the computer system and/or the data cloud storage stores at least one set of reference data representing at least one quality parameter associated to at least one type of agricultural group and/or type of agricultural group location and suitable for being applied for reference data for determining the at least one quality parameter for the respective type of agricultural group and/or type of agricultural group location.

The quality parameter is a quantity parameter or a derived quantity parameter, preferably selected from a parameter of quantity of the biological element, a parameter of quantity of the biological element at a selected time from an initial event, a parameter of quantity of the biological element relative to a threshold, a parameter of quantity of the biological element relative to a quantity of one or more other biological elements and/a derived parameter of one or more of these. The initial event may be as described below.

The data cloud storage may comprise data for the operation of the quality system and/or data collected and/or generated by the quality system may be stored in the data cloud storage. The data cloud storage may be provided by any data cloud storage systems and/or provider, such as a data cloud storage that runs on a cloud computing platform and may be accessed via the internet.

In an embodiment, the system is configured for storing data representing the determined quality parameter of the agricultural group, such as the flock of animal associated to event data representing the agricultural group and the agricultural group location. The data may be transmitted to the data cloud storage and stored or where the computer system form part or is integrated with the data cloud storage the data may simply be stored on the computer system.

In an embodiment, the system is configured for storing data representing the determined quality parameter of the agricultural group associated to event data representing the agricultural group and the feed agricultural group location and optionally to transmitting this data to the data cloud storage as event data sets.

Advantageously, each event data set represents a determined quality parameter and its associated event data. The event data may be data representing any kind of event, such as birth of animal(s), death of animal(s) (mortality rate), starting up of a new flock of animals, starting up of egg laying of a flock of animals, outbreak of a disease and/or illness of one or more animals of the flock of animals, special weather condition (very hot/very cold/storm/ high moisture etc.) and/or any other event that may be relevant to a farmer and/or feed dealer. The event may advantageously be an initial event as described elsewhere herein

By associating the quality parameter to an event, such as an initial event and thereby the event data representing the agricultural group and the agricultural group location preferably as a function of time relative to the event, a highly improved system for fast determination has been achieved. The very fast determinations provides improved possibilities for fast optimization of the quality.

In an embodiment, wherein the quality parameter is a quality parameter of a flock of animals and the quality parameter is a biological parameter, a health parameter, a growth parameter and/or a meat quality parameter, a production parameter. In an embodiment, wherein the quality parameter is quality parameter of a feed lot and/or an animal bedding lot and the quality parameter is a durability parameter, crispness parameter, a palatability parameter, a chemical parameter and/or biological parameter.

The biological parameter may for example be a parameter associated to a microbiological balance and/or an increase of one or more pathogens in the air, such as in particles e.g. from duct or from the animals, indicating the emergence of a disease.

It has been found that by applying biological element determinations for determining the quality parameter, a very fast and accurate quality determination may be obtained. For example the quality parameter of a flock of animal may be determined much earlier than what may be observable by monitoring the general physical status of the animals of the flock of animals, such as monitoring of the behavior or activity of the animals. Thus a change e.g. a decrease of quality parameter may be determined even before the animals of the flock of animals becomes physically effected of the change of quality parameter.

Particles in the air may be temporarily airborne, e.g. due to animals and/or farmers movements at the agricultural group location.

In an embodiment, the biological parameter comprises alfatoxin B1 level, bacterial and fungal counts.

The health parameter may for example be a parameter indicating a health condition of one or more of the animals of the flock or a parameter indicating the level of protection from a vaccination.

The growth parameter may for example be a parameter associated to growth, such as weight gain of the animals, feed intake, water consumption, mortality rate, laying rate, live birth rate.

The meat quality parameter may for example be a parameter associated to a quality characteristic of the meat or eggs, such as color, fat content, conditions such as woody breast, damage to the food, presence of zoonotic pathogens.

The production parameter may for example be a parameter associated to the production of milk, eggs, fur, weight gain etc.

The chemical parameter may for example be peroxide value or thiobarbituric acid value, gas levels (CO2, O2, NH4, etc.).

In an embodiment, the computer system and/or the data cloud storage stores data representing at least one quality parameter associated to at least one type of animal location and/or type of flock of animals or type of feed lot location and/or type of feed lot for which the at least one quality parameter is determinable. Thereby the biological element determinations performed may be associated to a type of flock of animals, a type of animal location, a type of feed lot location and/or a type of feed lot.

The quality system may advantageously be adapted for determining two or more quality parameters, such as at least three quality parameters, such as at least 4 quality parameters. These two or more quality parameters may advantageously be determined based on the same collected particles. It has been found that the amount of particles required for performing the biological element determination are surprisingly small, enabling several biological element determination to be performed on the same collection of particles.

Thereby the system becomes very cost effective, since several different sensors are not required.

In an embodiment, at least one quality parameter is selectable by the user. The computer system may advantageously have an interface to a user, e.g. via a screen and/or an audible interface where the user may select the quality parameter to be determined.

In an embodiment, the quality system is configured for selecting and/or suggesting the at least one quality parameter. The quality system may for example be configured for selecting and/or suggesting the at least one quality parameter at least partly in dependence on at least one of type of animal location, type of flock of animals, type of animal bedding lot, type of animal bedding lot location, type of feed lot and/or type of feed lot location. For example, if the user feed the data of type of agricultural group and/or agricultural group and/or at least one agricultural group location to the computer system via the interface and the computer system may select and/or suggest one or more quality parameters to be determined optionally in addition to the quality parameter(s) selected by the user.

If, for example the computer system and/or the data cloud storage comprises data representing an event, such as outbreak of a condition, such as an illness , poor quality of feed, low production or other undesired qualities of a flock of animals and/or a feed lot at a location of same or similar type and/or where the flock of animals and/or feed lot is/are of same or similar type the computer system may suggest or select one or more quality parameter(s) associated to the condition in question.

In an embodiment, the quality system is configured for selecting event data sets from the data cloud storage, which event data sets each comprises data representing a determined quality parameter associated to at least one type of animal location and/or type of flock of animals or type of feed lot and/or type of feed lot location wherein at least one of the flock of animals and/or its animal location or the feed lot and/or the feed lot location corresponds to the type of animal location and/or type of flock of animals or type of feed lot and/or type of feed lot location.

In an embodiment, the quality system is configured for selecting the event data sets such that each event data set comprises data representing a determined quality parameter, wherein the determined quality parameter is outside a threshold, such as outside a drift threshold and/or a value threshold.

Thereby the system may identify a possible undesired event(s) which may trigger an undesired development, such as an unbalance, an infection, a reduction in quality etc. The system may then suggest that the one or more quantitative biological element determinations performed comprises one or more quantitative biological element determinations for performing determination of the quality parameter in question.

The selected event data sets may be applied as the reference data.

The term threshold is herein used to mean an upper level, an upper level as a function of time a lower level, a lover level as a function of time, a level of changes (for example changes up and down over time), drift or any combinations comprising one or more of these.

In an embodiment, the quality system is configured for selecting the event data sets such that each event data set comprises data representing a determined quality parameter, wherein the determined quality parameter is exceeding a threshold or is below a threshold or is outside a threshold range for a value or a drift or any other relevant parameter describing the quality parameter.

In an embodiment, the system is configured for selecting and/or suggesting the at least one quality parameter and there by the one or more quantitative biological element(s) determinations to be performed to determine the quality parameter, at least partly in dependence on event data sets of the data cloud storage.

In an embodiment, the computer system and/or the data cloud storage stores data representing instructions for collecting particles from air. Preferably the data representing instructions for collecting particles from air comprises a database of collecting instructions correlated to at least one type of agricultural group and/or agricultural group location

The instructions for collecting particles from air may advantageously be displayed for the user. The user may for example feed the data of type of animal location, type of flock of animals, type of feed lot and/or type of feed lot location to the computer system via the interface and the computer system may then display the instructions for collecting the particles.

The type of agricultural group location may be any location suitable, therefore.

Specification of the type of animal location advantageously comprises information on at least one of
- outdoor or indoor or part of both, if at least partly indoor, advantageously, height to ceiling
- size e.g. total size, size per animal or a classified size range
- geographical location e.g. country or latitude/ longitude
- proximitry to other animal flocks (may be derived from geographical location) .

Specification of the type of flock of animals advantageously comprises information on at least one of
- single species/multi species
- species identification(s)
- number of animals e.g. approximately number or a classified number (e.g. class 1 for 1-10, class 2 for ....etc.)
- age, e.g. average age or number in various class of age
- production form (tradional, organic, free range etc.
- breeding/non-breeding
- dairy or egg producing/non- dairy/egg producing
- time to slaughtering
- vaccination history
- animal density (e.g. weight of animal per square meter animal location
- history from previous flocks (e.g. disease history growth history etc.).

Specification of the type of feed lot location and/or animal bedding lot location advantageously comprises information on at least one of
- outdoor or indoor or part of both, if at least partly indoor, advantageously, height to ceiling
- size e.g. total size, size per feed volume/mass unit or a classified size range
- geographical location e.g. country or latitude/ longitude
- on board a transportation unit (truck, ship) or not.

Specification of the type of feed lot advantageously comprises information on at least one of
- feed type(s) (wet/dry/protein content/)
- type of animal it is adapted for,
- volume/mass of lot.
- producer
- components and their origin and transport route.

Specification of the type of animal bedding lot advantageously comprises information on at least one of
- bedding type(s) (wet/dry/protein content/)
- type of animal it is adapted for,
- volume/mass of lot.
- producer
- components and their origin and transport route.

In an embodiment, the computer system is configured for receiving input data representing type of agricultural group and/or type of agricultural group location and for selecting collecting instructions and/or modified collection instructions and display these visually and/or audibly.

The collecting instructions advantageously comprises instructions of time slots for collecting particles, instructions of collecting length of time for collecting, instructions of location and/or movement of collector relative to agricultural group.

The time slots may be a specific time or a time range.

In an embodiment, each the time slots are specific time this specific time may be applied as the "time of collection" and may be represented by the time of attribute. The setting of the time of collection may for example be the time of starting the collection or the time of terminating the collection or a time there between, wherein the same setting of time of collection is applied for all times of collections.

Advantageously the time slot - preferably each of the time slots - is a time range in which the user may perform the collection. This provides flexibility for the user that the time of collection may be selected within the time slot given.

Where a time slot is a range of time in which the user may perform the collection, the "time of collection" is advantageously the actual time of collection, such the actual time of starting the collection or the actual time of terminating the collection or a time there between, wherein the same setting of time of collection is applied for all times of collections. For example a slot of collection may be between 7:00 and 11:00. If the farmer then starts the collection at 8:45, the actual time of collection, here 8:45 may be applied as the "time of collection" and may be represented by the time of attribute.

Movement(s) of collector relative to animals of the flock of animals or relative to the feed of the feed lot and/re relative to the animal bedding of the animal bedding lot may for example designate a collection distance to the animal(s)/feed/bedding, speed of movement proximity to the animal(s)/feed/bedding. The collection instructions advantageously include moving the collector around in multiple sites of the animal location/feed lot location/bedding location

The animal(s) of the flock of animals may be any kind of animals, in particular animals used for husbandry.

The flock of animals may for example comprise livestock animals and/or poultry. In an embodiment, the flock of animals comprises cattle, sheep, pigs, goats, horses, insects, camels, donkeys, mules, poultry, turkeys, geese, ducks and/or minks.

Advantageously, the flock of animals is a single species flock of birds or mammals. The expression "single species" is herein used to designate same type of animals. When determine the animals and animal species of the flock of animas, insects and microorganism are disregarded except where the flock of animals is a flock of insects. Advantageously, also undesired pests, such as rats and mice are also not counted when determining the animals of the flock of animals.

Advantageously, preferably at least 90 % by number, such as at least 95 % by number, such as at least about 99 % by number such as all of the animals of the flock of animals are of single species of birds or mammals.

In an embodiment, the flock of animals is a plural species flock of birds and/or mammals, such as a flock of animals comprising two species, 3 species, 4 species or at least 5 species of birds and/or mammals.

For example, the flock of animals may in an embodiment comprise horses and pigs or cattle and ducks.

The number of animals of the flock of animals is at least two, but usually the number will be higher, such as at least 5, such as at least 10, such as at least 30, such as at least 50, such as at least 100 or even more especially where the animals are relatively small, the number of animals of the flock may be very large, even up to several thousands or millions e.g. where the animals are insects.

Examples of flocks of animals includes the following:
In an embodiment, the flock of animals comprises a flock of insects, such as silkworms, mealworms buffaloworms (and optional beetles producing the larvae), honeybees and optionally commodities, lac insects, cochineal, crickets, cockroaches or waxworms (and optionally wax moths producing the larvae).

In an embodiment, the flock of animals comprises a flock of Black soldier flies and/or larvae e.g. from wax moth.

Where the flock of animals comprises insects the collecting instructions may for example comprise instructions to collect samples from air that has passed through the insect population.

In an embodiment, the feed lot comprises feed for mammals and/or birds and/or insects, the feed lot advantageously comprises feed for livestock animals and/or poultry, preferably cattle, sheep, pigs, goats, horses, donkeys, mules, chickens, turkeys, geese ducks and/or minks.

In an embodiment, the feed lot comprises feed for Aquatic animals, such as for crabs, fish, seals, octopus, snails, eels or clams. The feed lot may for example comprises dry forage, roughages, pasture, green forages, silages, pellets and/or granulates.

In an embodiment, the feed lot comprises energy feeds; protein supplements; vitamin supplements; and mineral supplement

In an embodiment, the animal location and/or the animal bedding location comprises an outdoor location such as a field, such as a field bounded by hedges and/or fences.

In an embodiment, the animal location and/or the animal bedding location comprises an at least partly indoor location, such as a barn, a cage, a house, a shed, a shelter and/or a shelter cover.

The feed lot location and/or animal bedding lot location may for example be a production site, at or in a transportation unit, a storing space, such as a container, a sac or a silo.

The transportation unit may e.g. be a truck, a ship.

The animal bedding advantageously comprises straw, sawdust, hemp straw, fleece, sand, wood shaving, peanut hulls, shredded sugar cane, peat moss or mixtures comprising any of these.

In an embodiment, the particles are collected from the animal location at an event comprising changing the litter e.g. in a barn. It has been found that biological element e.g. pathogens or similar at a very early stage may be found e.g. attached to dust in the litter and then may be "airborne" during the change.

The analyzing system may be configured for receiving the plurality of samples, one at a time or simultaneously. Advantageously, the system is configured for receive the samples one at a time, such as in the consecutive order as the particles for the respective samples are collected. Thereby, the development of the quality parameter may be followed over time and any negative changes may optionally be countered at an early stage, thereby improving the final level of the quality parameter.

The analyzing system is configured for receiving the plurality of samples in the form of chambers, such as sample chambers, comprising the samples or collected particles for the samples. Thereby the transfer of the collected particle becomes very simple and safe, and at the same time the risk of contaminating the collected particles becomes very low.

A sample of particles may be the total collected fraction of particles or a fraction thereof. In an embodiment, at least one of the samples, preferably each sample is the total amount of collected particle at one of the time slots. In an embodiment, at least one of the samples, preferably each sample is a volume or weight part of the total amount of collected particle at one of the time slots. In an embodiment, at least one of the samples, preferably each sample is a fraction comprising microorganism sampled of collected particle at one of the time slots and/or is a fraction comprising nucleic acid sequences extracted from collected particle at one of the time slots.

In an embodiment, at least one of the time slots, such as each time slot is equal to the time of collecting particles for a sample. In an embodiment, at least one of the time slots, preferably each time slot is longer than the time of collecting particles for a sample. By providing that the time slots are longer than the collection, the user may have the possibility of selecting within the time slot, when to collect the particles. This may be very convenient for the user to have this degree of freedom. However, it should advantageously be ensured that the time slot is not too long, since this may reduce the accuracy of the determined quality parameter.

Advantageously, at least one of the time slots, preferably each time slot has a length of more than 1 minute, such as more than 10 minutes, such as from 1 - 8 hours, such as from 1 to 24 hours, such as from 1 to 30 days, such as from 1-10 weeks.

The minimal length of the time slot is the length of the collection time. The actual time of collection i.e. the tile used for the time attribute may be set to be the start of collection or the termination of the collection or any selected time there between maximal length of the time slot may advantageously be selected in dependence of the type of animal location and/or type of flock of animals or type of feed lot and/or type of feed lot location. In certain situation the time slot may be very long such at one week, one month or even one year. This may for example be relevant for determination of biological parameter(s) associated to quality parameters(s) linked to fertility status and/or maturity status.

Advantageously, the consecutive, selected time slots are not overlapping, since this may result in reducing the accuracy of the quality parameter determination.

In an embodiment, the consecutive, selected time slots have time interval(s) between slots, which are preferably at least as long as each of a previous and subsequently time slot. Advantageously, time interval(s) between time slots is at least 2 times as long as each of a previous and subsequently time slot.

In an embodiment, the consecutive, selected time slots are defined by a minimum and/or a maximum time interval between start time and/or end time of respective particle collections.

The slots may for example be correlated to a life cycle of the flock of animals, to an average/medium age of the animals of the flock and/or to a time of year or day. For example the length of time slots or the time intervals between time slots may change along the life cycle of the flock of animals e.g. such that the time slots becomes shorter and more frequent as the animals approaches a time of breeding, time of moving to a different location, or a time of slaughtering or any other selected event.

In an embodiment, the time slots are selected to start at a specified time after a previous time of collecting, i.e. the time intervals between time slots may not have a specified length but instead a maximal length or a minimum length.

The analyzing system may be any analyzing system capable of performing the biological element determination. In an embodiment, the analyzing system comprises laboratory equipment adapted for at least partly performing the at least one quantitative, biological element determination of each of the received samples.

In an embodiment, the analyzing system comprises an analyzer configured for at least partly performing the at least one quantitative, biological element determination of each of the received samples. The analyzer may e.g. be an automatic analyzer, such as a robot

In an embodiment, the analyzer is configured for performing a PCR amplification, sequencing, mass spectroscopy, high pressure liquid chromatography, incubation, microbiological examination and/or optical identification.

Advantageously, the system comprises a collector adapted for collecting airborne particles.

The collector is advantageously a mobile collector, such as a handheld collector.

Examples of suitable collectors comprises an electrostatic collector and/or a filter collector.

Advantageously, at least a part of the analyzing system is integrated with the collector, preferably at least a part of the analyzer is integrated with the collector, optionally the entire analyzer is integrated with the collector.

The analyzer may e.g. be as the analyzer as described in any one of EP1730519, EP1730518 and EP1730276 or any modified or improved analyzer. For example, the collector may be configured such that the collection chamber may be withdrawn from the collector for simple transportation to the analyzer.

In a preferred embodiment, the computer system is configured for receiving the consecutively sub-sets of the data in real time as they are generated by the analyzing system. Thereby, the development of the quality parameter(s) may be followed, and undesired development may be counteracted at an early stage of development.

Advantageously, the computer system is configured for correlating the received consecutively sub-sets of the data with the reference data in real time as they are received by the computer system and preferably for, for each of the correlations with reference data, performing the determination of the quality parameter of the flock of animals and/or the quality parameter of the feed lot. Thereby any undesired change or indication thereof may be discovered at a very early stage. For example, an unbalance between two otherwise harmless biological elements may reduce resistance of animals of a flock or of the feed, thereby increasing risk of being infected by an undesired pathogen.

Advantageously, the computer system and/or the data cloud storage stores at least one set of reference data. Preferably the sets of reference data each comprises reference data representing quantity of the biological element as a function of time correlated to the quality parameter in question.

In an embodiment, the computer system and/or data cloud storage stores at least one set of reference data, correlated to at least one type of agricultural group and/or type of agricultural group location.

The stored set(s) reference data may for example be obtained or obtainable by being programmed to the computer system and/or by being acquired by machine learning and/or by being uploaded by previous users.

New sets of reference data may be uploaded to the data cloud storage e.g. on a regularly basis, or continuously e.g. by users. In an embodiment, the computer system is adapted to select desired data sets, such as a series of sub-sets of data obtained by users and data representing the associated quality parameter and to upload these assets of reference data

In an embodiment, the quality parameter of the stored set(s) reference data is obtained or is obtainable by determining the quality parameter as an actual quality determined by analysis of at least one animal of the flock of animals or of at least one fraction of the feed lot. The series of sub-sets of data obtained by user and the associated actual quality may thereafter be stored as a set of reference data.

In an embodiment, data representing the determined quality parameter and the associated sub-sets of the data received by the computer system is stored as at least a part of a set of reference data.

In an embodiment, data of sub-sets associated to a determined quality parameter and data representing an actual quality determined by analysis of at least one animal of the flock of animals or of at least one fraction of the feed lot is stored as at least a part of a set of reference data.

Advantageously, the set of reference data comprises data representing the associated type of animal location and/or type of flock of animals and/or type of feed lot and/or type of feed lot location.

In an embodiment, at least one of the stored set(s) reference data comprises data representing the at least one threshold quality parameter in the form of a threshold quantity and/or threshold drift of quantity of the at least one biological element as a function of time correlated to a selected level of the quality parameter.

The threshold quality parameter may be as described above.

Advantageously, the threshold(s) is/are generated by the computer system based on the set(s) of reference data. The threshold may advantageously be updated or modified when additional set(s) of reference data is/are stored in the computer system and/or the data cloud storage.

Advantageously, at least one of the stored set(s) reference data represents at least one threshold change of quantity of the biological element as a function of time correlated to a selected level of the quality parameter.

In an embodiment, at least one of the stored set(s) reference data represents at least one threshold change of quantity of the biological element as a function of time from an initial event correlated to a selected level of the quality parameter. The initial event may preferably be as the event(s) described above. In an embodiment, the initial event is hatching, birth, vaccinating, medicating, detection of disease related pathogens, movement of animals, change of light setting, feed change, and/or outbreaks in neighboring herds.

For example, if an outbreak e.g. of an undesired microorganism is discovered in a neighboring herd, the quality system may suggest or select a correlated quality parameter to be monitored and/or modify the collecting instructions making the time slots more frequent, and/or the quality system may modify the threshold and/or use set(s) of reference data from the neighboring herd as main reference data. In an embodiment, the quality data from the system may be compared with that from the neighbor and/or other previous outbreaks.

In an embodiment, at least one of the stored set(s) reference data represents the at least one threshold quality parameter derived of a relation of quantity of two or more biological elements correlated to a selected level of the quality parameter.

In an embodiment, the level of the quality parameter, is a level where the quality parameter is desired, a level where the quality parameter is undesired, is a level where the quality parameter indicates a raised risk, such as a risk of low production, low growth, of undesired infection, of increase in mortality rate, of decay of feed lot, a zoonotic risks and/or any other raise in risk of decrease in quality.

The computer system may be configured for determining a diagnostic indication of the agricultural group based on the determined quality parameter, when the determined quality parameter is preferably outside or inside a threshold range and/or where the quality parameter is exceeding a threshold.

In an embodiment, the computer system is configured for determining a diagnostic indication that one or more of the animals of the flock of animals may be infected based on the determined quality parameter, in case the determined quality parameter is outside a desired level, such as outside a threshold. The diagnostic indication may advantageously be confirmed by a veterinarian.

In an embodiment, the computer system is configured for determining a treatment indication for treating the flock of animals of the feed lot based on the determined quality parameter, in case the determined quality parameter is outside a desired level. Where the treatment indication is for treating the flock of animals, the treatment indication may advantageously be confirmed by a veterinarian.

The computer system may for example be configured for determining a treatment indication based on the determined quality parameter relative to the threshold.

In an embodiment, the computer system, is configured for determine a diagnosis, an indication of a diagnosis, a treatment and/or an indication of a treatment for improving the state of the agricultural group based on the determined quality parameter, when the determined quality parameter is exceeding a threshold.

In an embodiment, the computer system is configured for determine a diagnosis, an indication of a diagnosis, a treatment and/or an indication of a treatment for improving the state of the flock of animals with respect to the quality parameter. The computer system is advantageously configured for performing the determination of a diagnosis, of an indication of a diagnosis, of a treatment and/or of an indication of a treatment for improving the state of the flock of animals with respect to the quality parameter based on the determined quality parameter, and preferably in case where the determined quality parameter is outside a desired level, such as a threshold.

In an embodiment, the at least one set of reference data comprises the threshold in the form of at least one threshold change of quantity of the biological element as a function of time from an initial event.

Where the agricultural group is a flock of animals and the agricultural group location is an animal location, the initial event may advantageously be selected from hatching, birth, vaccinating, medicating, detection of disease related pathogens, movement of animals, change of light setting, feed change, and/or outbreaks in neighboring herds.

Where the agricultural group is a feed lot and the agricultural group location is a feed lot location and/or the agricultural group is an animal bedding lot and the agricultural group location is an animal bedding lot location, the initial event may advantageously be selected from a change of temperature, change of location, change in humidity, change of season, mixing with another feed lot and/or breach of a biosecurity protocol, such as a standard health protocol which documents procedures necessary for maintaining animal health of a flock of animals.

The invention also comprises a method of determining a quality parameter of a flock of agricultural group located at an agricultural group location, the method comprising
- providing a plurality of samples, each sample comprises particles or fragment(s) of particles collected from air from the agricultural group and/or at least one agricultural group location at consecutive, selected time slots wherein each sample being correlated with a time attribute representing a time of collection;
- performing at least one quantitative, biological element determination of each of the samples to obtain data sub-sets comprising for each sample the result of the at least one determination and the time attribute for the sample.
- correlating one or more of the data sub-sets with reference data comprising at least one set of reference data; and
- determining the quality parameter of the agricultural group
wherein the set of reference data represents reference quantity of the biological element as a function of time correlated to the quality parameter comprising at least one threshold quality parameter of the at least one biological element as a function of time and wherein the determination of the quality parameter comprises determining quantity of the at least one biological element as a function of time and wherein the method further comprises determining the quality parameter relative to the at least one threshold quality parameter.

The agricultural group and agricultural group location may be as described above.

In an embodiment, the method comprises determining a quality parameter of a flock of animals located at an animal location combined with a method of determining a quality parameter of a feed lot located at a feed lot location and/or location combined with a method of determining a quality parameter of a bedding lot located at a bedding lot location. The feed lot may for example comprise feed for the animals of the flock of animals and/or the animal bedding lot ay for example comprise bedding material for the animals of the flock of animals.

The method may advantageously be performed using a quality system described above.

The quality parameter and the threshold quality parameter may be as described above.

Advantageously, the quality parameter is quality parameter of a flock of animals and the quality parameter is a biological parameter, a health parameter, a growth parameter and/or a meat quality parameter, a production parameter or wherein the quality parameter is quality parameter of a feed lot and the quality parameter is a chemical and/or biological parameter, which may for example be selected by the user e.g. together with the associated quantitative, biological element determination.

The method may comprise determining two or more quality parameters, such as at least three quality parameters, such as at least 4 quality parameters.

In an embodiment, the method comprises selecting the at least one quality parameter at least partly in dependence on type of the animal location, type of the flock of animals, type of the feed lot and/or type of the feed lot location.

Advantageously, the quality parameter is a quantity parameter or a derived quantity parameter, preferably selected from a parameter of quantity of the biological element, a parameter of quantity of the biological element at a selected time from an initial event, a parameter of quantity of the biological element relative to a threshold, a parameter of quantity of the biological element relative to a quantity of one or more other biological elements and/a derived parameter of one or more of these.

The particles may advantageously be collected as described e.g. using a method comprising following predetermined collecting instructions, such as the collecting instructions described above e.g. with time slots as described above.

Advantageously, the at least one quantitative, biological element determination is performed by a method comprising a PCR amplification, a sequencing, a mass spectroscopy procedure, a high pressure liquid chromatography procedure, an incubation procedure, a microbiological examination and/or an optical identification, wherein the sample advantageously may be as described above.

In an embodiment, the reference data comprises at least one set of reference data, correlated to at least one type of agricultural group location and/or at least one type of agricultural group The reference data may be as and be generated as described above.

Advantageously, the reference data, such as the set(s) of reference data represents at least one threshold quantity and/or threshold drift of quantity of the at least one biological element as a function of time correlated to a selected level of the quality parameter. The threshold may be as described above.

Advantageously, the reference data, such as the set(s) of reference data represents at least one threshold change of quantity of the biological element as a function of time from an initial event correlated to a selected level of the quality parameter, the initial event may preferably be hatching, birth, vaccinating, medicating, detection of disease related pathogens, movement of animals, change of light setting, feed change, and/or outbreaks in neighboring herds.

Advantageously, the method comprises determining if the quality parameter indicates a raised risk, such as a risk of low production, low growth, of undesired infection, of increase in mortality rate, of decay of feed lot, a zoonotic risks and/or any other raise in risk of decrease in quality.

In an embodiment, the method comprises determining a diagnostic indication based on the determined quality parameter, when the determined quality parameter is outside a desired level, such as outside a threshold.

In an embodiment, the method comprises determining a treatment indication based on the determined quality parameter, when the determined quality parameter is outside a desired level.

### BRIEF DESCRIPTION OF THE EXAMPLES AND DRAWING

The invention is being illustrated further below in connection with selected examples and embodiments and with reference to the figures. The figures are schematic and may not be drawn to scale.
Figures 1a and 1b are diagrams showing a quantitative biological element determination for particles collected from animal locations of flocks of hens as a function of time.
Figure 2 is a diagram showing the determined quantity of the 4 biological element as a function of time.
Figure 3 is a diagram showing score of health of the digestive system of a as a function of time.
Figure 4 is a diagram associated to example 4 and showing Vaccine concentration in air determined from particles collected as described above as a function of time.
Figure 5 illustrates an embodiment of a quality system for determining a quality parameter of a flock of animals and/or a food lot.
Figure 6 illustrates a process diagram of an embodiment of the method and the quality system.
Figure 7 is a diagram associated to example 7 and showing Vaccine concentration in air determined from particles collected as described above as a function of time.
Figures 8a and 8b are diagrams associated to example 8 and showing Vaccine concentration in air determined from particles collected as described above as a function of time.

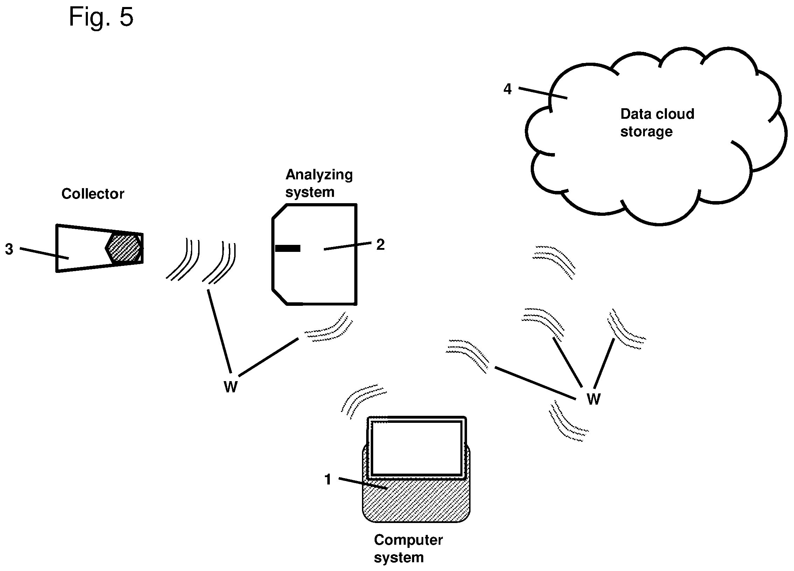

The quality system illustrated in figure 5, comprises a computer system 1, an analyzing system 2 and a collector. As indicated with the wave forms w, the computer system 1 is in data communication with a data cloud storage 4. The collector is in data communication with the analyzing system 2, Alternatively, the collector 3 and the analyzing system 2 may be integrated with each other. In addition the computer system is in data communication with the analyzing system 2. The computer system is here illustrated as a single computer, but as described above the computer system may comprise two or more computers in data communication. The data communication may advantageously be or comprise a wireless communication.

The collector 3 is adapted for collecting particles from an animal location and/or a feed location as described above. The collection is performed as described above at selected time slots. The time of collection is transmitted to the analyzing system 2. A sample of the collected particles are analyzed using the analyzing system 2 as described above and the result is paired with the time of collection to generate a sub-sets of data, each comprising data representing the at least one quantitative, biological element determination of a sample and the time attribute of the sample. The subsets are transmitted to the computer system 1 consecutively as generated or in blocks of sub-sets.

The computer system 1 is receiving at least one set of reference data from the data cloud storage 4, where the set of reference data represents quantity of the biological element in question as a function of time correlated to the quality parameter in question as described above. The set of reference data comprises at least one threshold quality parameter of the at least one biological element as a function of time.

The computer system 1 is correlating the received consecutively sub-sets of data with the set(s) of reference data as described above and perform a determination of the quality parameter of the at least one biological element as a function of time further comprises determines the quality parameter relative to the at least one threshold quality parameter.

The computer system 1 is advantageously configured for transmitting the sub-sets of data together with the determined quality parameter to the data cloud storage 4.

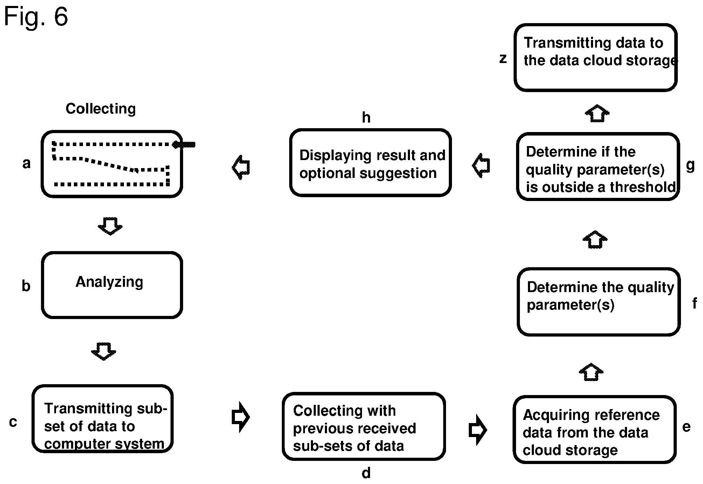

The process diagram illustrated in figure 6, illustrates a number of steps which may be repeated e.g. with variations in collection details, preferably according to collection instructions.

In step 1 particles are collected using a collector within a selected time slot and according to collection instructions as described above. A sample of the collected particles are analyzed in step b for performing at least one quantitative, biological element determination using an analyzing system.

The resulting determination is transmitted to the computer system in step c, as a sub-set of data also comprising a time attribute representing a time of collection

The sub-set of data received by the computer system is in step d collected with previous received sub-set of data if any.

In step e the computer system is acquiring reference data from the data cloud storage. Step e may be performed anywhere in the series of steps or it may be omitted if the computer system has already received reference data or stores reference data itself.

In step of the computer system determines the quality parameter as it is at the time of collecting the latest analyzed particles.

In step g the computer system determines if the quality parameter is outside a threshold.

In step h the computer system displays the result and optional suggestions e.g. as described above. And the steps are repeated. In the illustrated step z, the computer system transmits data comprising one or more sub-sets together with determined quality parameter(s) to the data cloud storage.

These steps may be omitted in one or more series of steps and/or it may be performed at any time in the series of steps.

### Example 1 - Correlations when monitoring of several biological elements

In this example concentration of a number of biological elements are determined at consecutive time slots to follow the development of these concentrations over time. The biological element are 7 different microbiological organisms (bacteria and virus). The time slots were 4 hours and the particle collection were performed once per month.

Surveillance of different microbiological organism may reveal an emerging infection spreading in a flock or herd. The surveillance program was conducted over a period of 6 months in 3 flocks of animals, namely three poultry houses with laying hens.

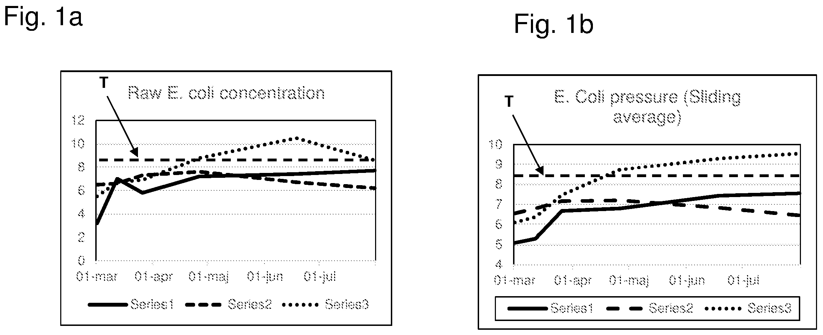

The quantitative recordings of one of the biological elements, namely E. coli in the three different poultry houses recorded over a period of 6 months is shown in figure 1a , where the series 1, represents poultry house 1, series 2 represents poultry house 2 and series 3, represents poultry house 3.

In two of the houses (poultry house 1 and poultry house 3) the concentration of E. coli in the air inside the houses continuously increases over time. Whereas, in the last house (poultry house 2) the concentration remained constant over time (it even showed a slight decrease). In the two houses where the concentration of the E. coli where increasing a wild strain of the infectious bronchitis virus was discovered in the air samples collected in June.

These measurements illustrate the use of the E. coli concentration as an indicator of the overall health of a poultry flock. In such a setup an increase in the E. coli concentration should encourage the producer to investigate the source of this increase, which may be both diseases related or metabolic related.

Processing of the data by using reference data may be advantageous for fast and accurate discovering a drop in a quality parameter especially because the quality parameter will vary naturally over time and the lifespan of the animals.

The raw E. coli data is shown in figure 1a, in which the natural short-term fluctuation of the pathogen pressure in the form of the quantity of the E. coli that to a certain extent will occur in the animal location, clouds the long-term tendencies of the E. coli pressure. A simple sliding average applied to the data as shown in figure 1b may limit the effects of the short-term variation and allow the long-term tendencies to become visible.

A threshold level "T" is illustrated in both figure 1a and figure 1b. This threshold level "T" is obtained from poultry houses of same type, i.e. similar size, number of and/or age of poultry.

As it can be seen on both figure 1a and figure 1b, the E. coli concentration in poultry house 2 was increasing the threshold level "T" already in early May. Thus had the system of the invention been applied, the farmer would have been alerted already in early May that an undesired level of E. coli was under development and likely the wild strain of the infectious bronchitis virus would have been found several weeks earlier.

In a first variation thereof, a threshold level "T" may be determined by professionals preferably base on their knowledge of which level supports fast growth and which do not, which level supports poultry well-being and which do not.

In a second variation thereof, a threshold level "T" may be obtained by historical data from the present farm and other like farms combined with information on other quality parameters collected from these farms at the same time. From these historical data it may be possible to derive the level of for instance E. coli in the poultry houses were the other quality parameters typically is also good. In such a situation if the E. coli concentration raises above the threshold it is indicative that some of the other quality parameters will likely also soon start to change.

### Example 2 -The Health of the digestive system of a flock

In this example particles are collected in a hen house with a flock of hens (25.000 - 100.000 pr. house). The collection is performed once every 5th day in a time slot between 10:00 and 12:00. Collecting time is 5 minutes.

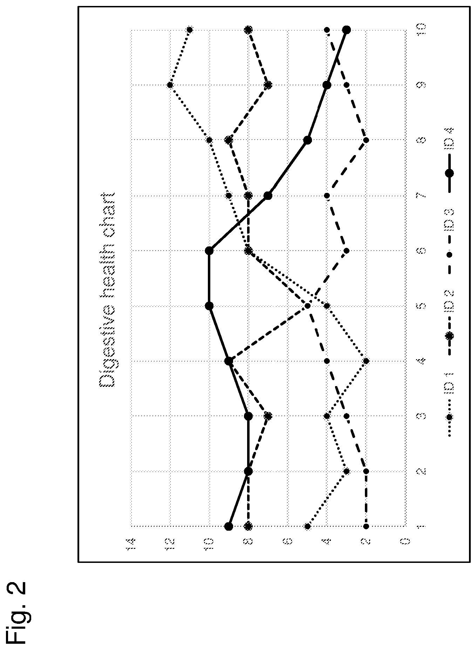

Quantitative determinations of 4 different biological elements are performed. The 4 different biological element are 4 different types of gut microbiota. The results are shown in figure 2.

Based on the relationship between the concentrations and the relative change over time the health of the digestive system of the flock can be monitored. In this way probiotic treatment may be initiated if some of the parameters drops, e,g. drops faster than a threshold or falls outside a threshold.

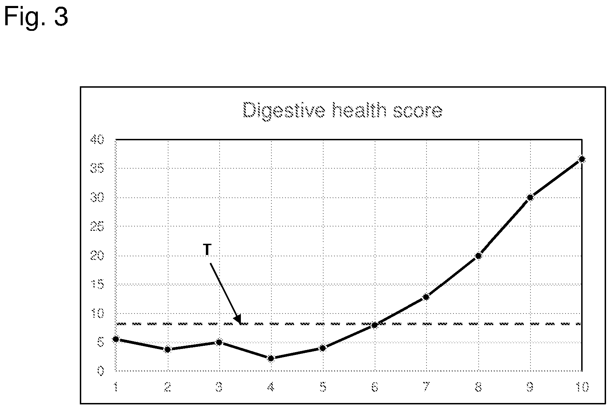

Figure 3 shows a comparison between the biological element with ID 1 and the biological element with ID 4. The biological element with ID 1 is believed to have positive effect on digestive health, whereas the biological element with ID 4 is believed to have negative. By dividing the biological element-ID 1 by the biological element - ID 4 a helpful quality parameter and a threshold therefor may be obtained.

In addition figure 3 illustrates digestive health scores with a simple value based reference threshold level "T". When the value break the threshold it indicates that there may be some underlying issue in the flock that should be further investigated either by traditional means or by analysis of curves from other quality parameters to provide an indication of the underlying issues.

By comparing the determined quantity of the 4 biological element with reference data, which may include threshold data, one or more quality parameter may be determined. The result may be communicated to the producers along with recommendations on which actions may be taken to increase the ratio and health of the flock and hence increase productivity of the production.

### Example 3 - Production Optimization

In this example a panel of different production related pathogens (biological elements), both directly and in directly disease related are determined from particles collected in an animal location as a function of time. Based on previous data analysis (reference data) both from the specific farm but also on data from other farms of same type of animal location and/or type of flock of animals in the area, country or even worldwide. Specific patterns in the change of concentration between these reference data are recognized so that the Al or numerical algorithm can inform that a rise in one of the pathogens is most likely followed by a rise in second pathogen and hence probiotic treatment may be initiated at a very early stage to avoid this risk.

### Example 4 - Vaccination protection and strategy

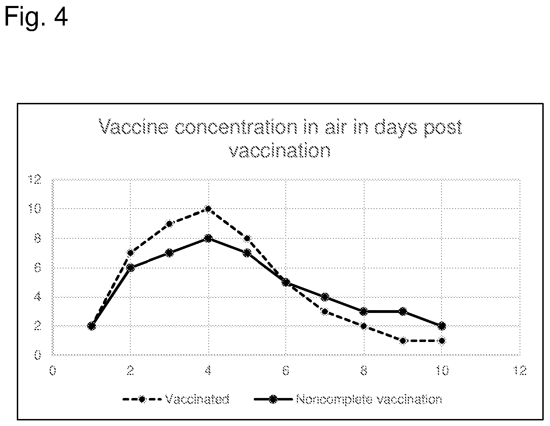

In this example the quantitative biological element determinations of particles collected over time as described above is used to validate that the vaccination procedure was conducted correctly and that the flock of animals is protected against the relevant disease. In such an application the biological element determined includes the live vaccine on the particles collected from the air inside the location comprising the flock of animals. The particles are collected at specific times according to given time slots post vaccination, to verify that the vaccine concentration in the air follows the expected trajectory indicating a successful vaccination procedure. Should only a minor fraction of the flock have received the vaccination, the remaining flock will remain naive and receive the vaccine from already vaccinated animals, and the concentration of the vaccine in the air, may remain elevated over longer times. Alternatively, if the vaccine does not spread to the naïve animals e.g. birds, but only a fraction of the birds received the vaccine the level may not exceed a threshold level, indicating that only a fraction of the population is prober protected. An Illustration of such situation can be seen in figure 4, where the noncomplete vaccinated flock excrete less of the vaccine in the peak days and the levels does not fall off as rapidly, as the case for the successfully vaccinated flock.

In a simple application the concentration of the vaccine on day 4 divided by the vaccine concentration on day 8 may provide the user with valuable information about the vaccination success.

### Example 5 - Vaccination protection and strategy

In a variation of example 4, the data generated by the quality system is used to qualify the decisions related to vaccination procedures in between flocks of animals or rotations of flocks of animals e.g. chicken in a broiler production. In such an application, vaccine may be given to the subsequent flock of animals at the animal location if any signs of an emerging disease are present in the current flock of animals. If signs show that for instance infectious bursal disease or infectious bronchitis virus is emerging in the current flock of animals, vaccination of the subsequent flock or flocks of animals may be initiated on an informed basis.

### Example 6 - Vaccination protection and strategy

Likewise, the data from the quality system may be used to ensure that the flocks of animals is healthy enough at the vaccination time so that the vaccination strategy may be successful. If for instance infectious bursal disease virus was present in the flock of animal e.g. birds at the time of vaccination, such an infection may affect the immune system of the birds. This may limit the effect of a vaccination procedure and hence may result in not sufficiently protected or even naive birds in the flock.

### Example 7 - Vaccination protection and strategy

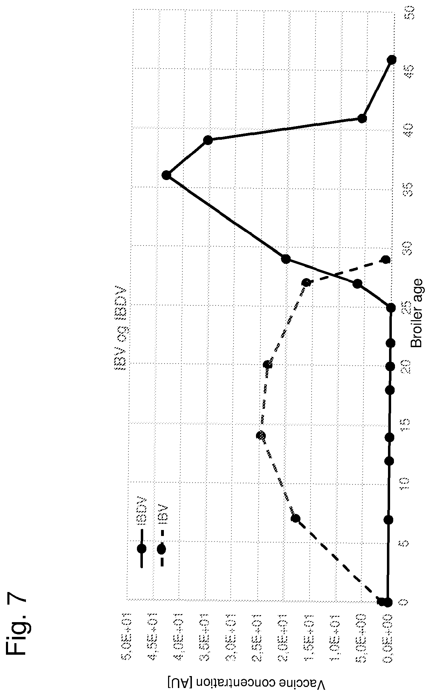

Chickens of a flock of chickens in a chicken house were vaccinated for infectious bronchitis virus (IBV) as one day old and for infectious bursal disease virus (IBDV) at day 20. In this example the vaccines were administered by spray and drinking water respectively. Air samples were collected throughout the production cycle as seen in figure 7. The vertical axis is a linear scale of the vaccine concentration in air samples of particles collected inside the poultry house and the horizontal axis is the age of broilers in days from IBV vaccination. The shape and the width of the bell shaped vaccine response curves can be used to derive the effectiveness of the vaccine. If for instance not all broilers was targeted by the original vaccine one would expect an extended curve. Furthermore the fact that the vaccine can be found in the air samples is also indicative that the vaccine reached the broilers and did in fact reproduce therein as intended - otherwise it would not be possible to find it in the air. Especially the fact that the vaccine is found in the air starting from 4-5 days post vaccination indicates that the vaccine in fact reached the broilers. If the vaccine was compromised by some means and lost its ability to infect the chickens and activate the immune response it would not be expected to see the vaccine in the air. Such a concentration in the air is only possible where the vaccine were reproduced in the broilers as intended.

In a variation of this example a number of the chickens, preferably at least 50 % of the chickens of the flock of chickens in a chicken house is vaccinated by injections.

### Example 8 - Vaccination protection and strategy

Chickens of flocks of chickens (broilers) in three chicken houses, house 1, house 2and house 3, were vaccinated for infectious bursal disease virus (IBDV) at day 20.

From day 11 particle samples were collected from each chicken house once every other day in a time slot between 10:00 and 12:00. Collecting time was about 10 minutes.

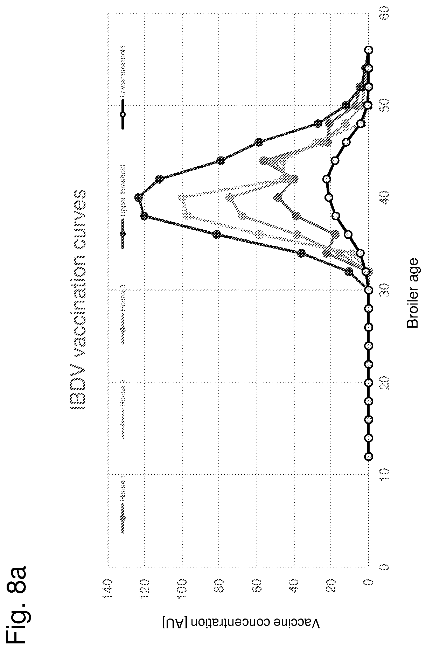

Determinations of vaccine concentration were determined from each sample. A plot of the results from chicken house 1, 2 and 3 are shown in Figure 8a.

Figure 8a also show upper and lower thresholds curves for the vaccine concentration.

This upper and lower thresholds curves for the vaccine concentration has been determined from measurements obtained from previous IBVD vaccinations both at the specific location and from other related locations i.e. chicken houses of same type (ie production form and race).

The lower threshold is set for ensuring an effective vaccination of the entire flock of chicken. If the vaccine concentration in a chicken house drops below this lower threshold, it indicates that the vaccine may not have been fully effective, for example if the distributed vaccine was compromised and did not work and/or a too low percentage of the chickens may have been sufficiently vaccinated to reach flock immunity. A supplementary vaccination may then be recommended.

The upper threshold is set for observing any undesired infections of the infectious bursal disease virus or presence of wild strains of infectious bursal disease viruses. As long as the vaccine concentration in a chicken house stays below the upper threshold, there is no need for further analysis (sequencing) to determine the virus variant or optional mutations.

If the vaccine concentration in a chicken house exceeds the upper threshold, this may indicate underlying issues in the house and or the vaccination procedure. Depending on when and how the threshold curves are broken it may be caused by different issues. If for instance vaccine curves are elongated or have a double top it may indicate that only a fraction of the birds were targeted by the initial vaccine and the remaining birds are affected from these birds. On the other hand If the vaccine curves follow the expected bell curve in the beginning but at the end starts to increase this may indicate that a wild strain is existing in the house and developing beneath the vaccine strain.

As it can be seen the upper and lower thresholds curves varies as a function of time from the time of vaccination.

The upper and lower thresholds curves may dynamically be updated from determinations of concentration of infectious bursal disease virus in related locations e.g. from determinations of concentration of infectious bursal disease virus obtained in other same type chicken houses in which the quality system according to the invention has been applied and where the determinations is uploaded to a data cloud storage e.g. as described above. The concentration determinations of the four chicken houses may in the same way be feed back into the system for updating the threshold data. This may e.g. be provide by uploading the determinations from the four houses to the data cloud storage.

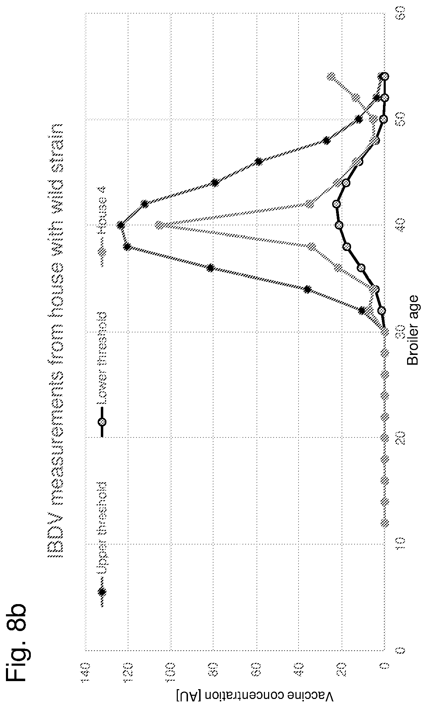

In figure 8b the results from chicken house 4 is plotted together with the upper and lower thresholds curves. Here it can be seen that around day 52 the determined of concentration of infectious bursal disease virus exceeds the upper threshold. Analysis showed that this was caused by a wild strain of infectious bursal disease virus. This information may be uploaded to the data cloud storage together with the determined of concentration of infectious bursal disease virus for house 4.

### Example 9 - Surveillance of a feed lot

The quality system is in this example used to monitor a feed lot comprising feed for animals. The feed lot may be monitored using the method described above at one or more feed lot locations in the production line e.g. at any point were the materials is handled and dust may be produced.

When importing goods by ship the entire load on the ships may be monitored at different locations during unloading to detect any presence of harmful or zoonotic pathogens present in the raw material feed lot to ensure proper handling at the production plant.

At the arrival at the feed production facilities the raw material feed lot may be monitored during unloading for the presence of the most common and/or harmful bacteria, virus, spores or other microbiological components to ensure that the harmful organism does not reach the farmers.

The feed lot may likewise be monitored in the mixing of the materials in the actual production of the feed lot. Particles may be at specific intervals or at specific times for instance during mixing and/or heating.

## Claims

1. A method of determining a quality parameter of an agricultural group located at an agricultural group location and preferably selected from a flock of animals, a feed lot and/or an animal bedding lot, the method comprising
- providing a plurality of samples, each sample comprises particles or fragment(s) of particles collected from air from said agricultural group location at consecutive, selected time slots wherein each sample being correlated with a time attribute representing a time of collection;
- performing at least one quantitative, biological element determination of each of said samples to obtain data sub-sets comprising for each sample the result of said at least one determination and said time attribute for said sample.
- correlating one or more of said data sub-sets with reference data comprising at least one set of reference data; and
- determining said quality parameter of the agricultural group,
**characterised in that**
said set of reference data represents reference quantity of said biological element as a function of time correlated to said quality parameter comprising at least one threshold quality parameter of said at least one biological element as a function of time and wherein the determination of said quality parameter comprises determining quantity of said at least one biological element as a function of time and wherein the method further comprises determining said quality parameter relative to said at least one threshold quality parameter,
wherein the set of reference data comprises event data representing the quality parameter of the agricultural group and the agricultural group location as a function of time relative to an initial event.

2. The method of claim 1, wherein said threshold quality parameter comprises at least one of
- a threshold quantity of said at least one biological element;
- a threshold drift of quantity of said at least one biological element as a function of time and/or
- a threshold change of quantity of said biological element as a function of time, optionally correlated to a selected level of said quality parameter.

3. The method of claim 1 or claim 2, wherein the quality parameter is a quantity parameter or a derived quantity parameter, preferably selected from a parameter of quantity of said biological element, a parameter of quantity of said biological element at a selected time from the initial event, a parameter of quantity of said biological element relative to a threshold, a parameter of quantity of said biological element relative to a quantity of one or more other biological elements and/or a derived parameter of one or more of these, preferably the method comprises determining two or more quality parameters, such as at least three quality parameters, such as at least 4 quality parameters.

4. The method of any one of claims 1-3, wherein the method comprises collecting said particles, preferably said particles being collected according to predetermined collecting instructions, said predetermined collection instructions preferably comprises instructions of time slots for collecting particles, instructions of collecting time, instructions of location and/or movement of collector relative to elements of the agricultural group, such as relative to animals of the flock of animals or relative to the feed of the feed lot or relative to the animal bedding of the animal bedding lot,
preferably the method comprises collecting said particles using a collector selected from an electrostatic collector and/or a filter collector.

5. The method of any one of claims 1-4, wherein said reference data, such as said set(s) of reference data represents at least one threshold change of quantity of said biological element as a function of time from the initial event correlated to a selected level of said quality parameter,
preferably said agricultural group is a flock of animals and said agricultural group location is an animal location, wherein said initial event is selected from hatching, birth, vaccinating, medicating, detection of disease related pathogens, movement of animals, change of light setting, feed change, and/or outbreaks in neighboring herds or
said agricultural group is a feed lot and said agricultural group location is a feed lot location and/or said agricultural group is an animal bedding lot and said agricultural group location is an animal bedding lot location and wherein said initial event is selected from a change of temperature, change of location, change in humidity, change of season, mixing with another feed lot and/or breach of a biosecurity protocol.

6. The method of any one of claims 1-5, wherein the agricultural group is an flock of animals and the agricultural group location is an animal location and wherein the quality parameter is a quality parameter of the flock of animals and the quality parameter is a biological parameter, a health parameter, a growth parameter and/or a meat quality parameter, a production parameter or wherein the agricultural group is a feed lot and said agricultural group location is a feed lot location, wherein the quality parameter is a durability parameter, crispness parameter, a palatability parameter, a chemical parameter and/or biological parameter.

7. A quality system for determining a quality parameter of agricultural group suitable for use in the method of any one of the preceding claims, the quality system comprising a computer system (1) in data communication with an analyzing system (2) adapted for analyzing samples of particles collected from air from an agricultural group location comprising the agricultural group,
wherein the analyzing system is configured
- for receiving a plurality of samples, each sample comprises particles or fragment(s) of particles collected from air from said agricultural group location at plurality of consecutive selected time slots and each sample being correlated with a time attribute representing a time of collection;
- for performing at least one quantitative, biological element determination of each of said received samples; and
- for transmitting sub-sets of data, each comprising data representing said at least one quantitative, biological element determination of a sample and the time attribute of said sample to said computer system;
wherein the computer system (1) is configured
- for receiving said sub-sets of data,;
- for correlating said received sub-sets of data with at least one set of reference data; and
- for determining said quality parameter of the agricultural group,
wherein said set of reference data represents reference quantity of said biological element as a function of time correlated to said quality parameter comprising at least one threshold quality parameter of said at least one biological element as a function of time and wherein the determination of said quality parameter comprises determining quantity of said at least one biological element as a function of time and wherein the computer system (1) further is configured for determine said quality parameter relative to said at least one threshold quality parameter,
wherein the set of reference data comprises event data representing the quality parameter of the agricultural group and the agricultural group location as a function of time relative to an initial event,
optionally the quality system comprises at least a part of said analyzing system.

8. The quality system of claim 7, wherein the computer system (1) and/or a data cloud storage (4) in data communication with said computer system (1) stores said at least one set of reference data, said sets of reference data each comprises reference data representing quantity of said biological element as a function of time correlated to said quality parameter, preferably said at least one set of reference data comprises reference data representing at least one quality parameter associated to at least one type of agricultural group and/or type of agricultural group location and suitable for being applied for reference data for determining said at least one quality parameter for said respective type of agricultural group and/or type of agricultural group location.

9. The quality system of claim 7 or claim 8, wherein the quality parameter is a quantity parameter or a derived quantity parameter, preferably selected from a parameter of quantity of said biological element, a parameter of quantity of said biological element at a selected time from the initial event, a parameter of quantity of said biological element relative to a threshold, a parameter of quantity of said biological element relative to a quantity of one or more other biological elements and/a derived parameter of one or more of these.

10. The quality system of any one of claims 7-9, wherein the computer system (1) and/or the data cloud storage (4) stores data representing instructions for collecting particles from air, preferably the data representing instructions for collecting particles from air comprises a database of collecting instructions correlated to at least one type of agricultural group and/or type of agricultural group location, preferably the collecting instructions comprises instructions of time slots for collecting particles, instructions of collecting time, instructions of location and/or movement of collector relative to animals of the flock of animals or relative to the feed of the feed lot.

11. The quality system of any one of claims 7-10, wherein the system comprises a collector (3) adapted for collecting at least partly airborne particles, wherein the collector is an electrostatic collector and/or a filter collector and wherein at least a part of the analyzing system (2) is integrated with the collector (3) to provide that the analyzer configured for at least partly performing the at least one quantitative, biological element determination of each of said received samples, preferably the analyzer is configured for performing a PCR amplification, sequencing, mass spectroscopy, high pressure liquid chromatography, incubation, microbiological examination and/or optical identification.

12. The quality system of any one of claims 7-11, wherein the computer system (1) is configured for receiving said consecutively sub-sets of said data in real time as they are generated by the analyzing system, wherein the computer system (1) is configured for correlating said received consecutively sub-sets of said data with said reference data in real time as they are received by the computer system (1) and preferably, for each of said correlations with reference data, performing said determination of said quality parameter of agricultural group.

13. The quality system of any one of claims 7-12, wherein said threshold is a selected level of said quality parameter, preferably said selected level of said quality parameter is a level where the quality parameter indicates a raised risk, such as a risk of low production, low growth, of undesired infection, of increase in mortality rate, of decay of feed lot, a zoonotic risks and/or any other raise in risk of decrease in quality.

14. The quality system of any one of claims 7-13, wherein said computer system (1) is configured for determining a diagnostic indication based on the determined quality parameter, when said determined quality parameter is preferably outside or inside a threshold range and/or where the quality parameter is exceeding a threshold,
preferably wherein said computer system, based on the determined quality parameter, when said determined quality parameter is exceeding a threshold, is configured for determine a diagnosis, an indication of a diagnosis, a treatment and/or an indication of a treatment for improving the state of the agricultural group.

15. The quality system of any one of claims 7-14, wherein said at least one set of reference data comprises said threshold in the form of at least one threshold change of quantity of said biological element as a function of time from the initial event and wherein said agricultural group is a flock of animals and said agricultural group location is an animal location, said initial event is selected from hatching, birth, vaccinating, medicating, detection of disease related pathogens, movement of animals, change of light setting, feed change, and/or outbreaks in neighboring herds.

16. The quality system of any one of claims 7-14, wherein said at least one set of reference data comprises said threshold in the form of at least one threshold change of quantity of said biological element as a function of time from the initial event and wherein said agricultural group is a feed lot and said agricultural group location is a feed lot location or said agricultural group is an animal bedding lot and said agricultural group location is an animal bedding lot location and wherein, said initial event is selected from a change of temperature, change of location, change in humidity, change of season, mixing with another feed lot and/or breach of a biosecurity protocol.

## Patentansprüche

1. Verfahren zum Ermitteln eines Qualitätsparameters einer sich an einem Standort einer landwirtschaftlichen Gruppe befindenden landwirtschaftlichen Gruppe, vorzugsweise ausgewählt aus einer Tierherde, einer Mastanlage und/oder einer Tierstreuanlage, wobei das Verfahren Folgendes umfasst
- Bereitstellung einer Vielzahl von Proben, wobei jede Probe Partikel oder Partikelfragment(e) umfasst, die aus Luft an dem Standort einer landwirtschaftlichen Gruppe in aufeinanderfolgenden, ausgewählten Zeitfenstern entnommen sind, wobei jede Probe mit einem Zeitattribut korreliert ist, das einen Entnahmezeitpunkt darstellt;
- Durchführen mindestens einer quantitativen Ermittlung biologischer Elemente mit jeder der Proben zum Erhalten von Datenteilsätzen, die für jede Probe das Ergebnis der mindestens einen Ermittlung und das Zeitattribut für die Probe umfassen;
- Korrelieren eines oder mehrerer der Datenteilsätze mit mindestens einem Satz von Referenzdaten umfassenden Referenzdaten; und
- Ermitteln des Qualitätsparameters der landwirtschaftlichen Gruppe,
**dadurch gekennzeichnet, dass** der Satz von Referenzdaten eine Referenzmenge des biologischen Elements als Funktion von Zeit darstellt, die mit dem Qualitätsparameter korreliert ist, der mindestens einen Schwellenwert-Qualitätsparameter des mindestens einen biologischen Elements als Funktion von Zeit umfasst, und wobei die Ermittlung des Qualitätsparameters Ermitteln einer Menge des mindestens einen biologischen Elements als Funktion von Zeit umfasst und wobei das Verfahren ferner Ermitteln des Qualitätsparameters relativ zu dem mindestens einen Schwellenwert-Qualitätsparameter umfasst,
wobei der Satz von Referenzdaten Ereignisdaten umfasst, die den Qualitätsparameter der landwirtschaftlichen Gruppe und den Standort einer landwirtschaftlichen Gruppe als Funktion von Zeit relativ zu einem Anfangsereignis darstellen.

2. Verfahren nach Anspruch 1, wobei der Schwellenwert-Qualitätsparameter mindestens eines der Folgenden umfasst
- eine Schwellenwertmenge des mindestens einen biologischen Elements;
- einen Schwellenwertdrift der Menge des mindestens einen biologischen Elements als Funktion von Zeit und/oder
- eine Schwellenwertänderung der Menge des biologischen Elements als Funktion von Zeit, optional korreliert mit einem ausgewählten Niveau des genannten Qualitätsparameters.

3. Verfahren nach Anspruch 1 oder Anspruch 2, wobei der Qualitätsparameter ein Mengenparameter oder ein abgeleiteter Mengenparameter ist, vorzugsweise ausgewählt aus einem Parameter der Menge des biologischen Elements, einem Parameter der Menge des biologischen Elements zu einem ausgewählten Zeitpunkt nach dem Anfangsereignis, einem Parameter der Menge des biologischen Elements relativ zu einem Schwellenwert, einem Parameter der Menge des biologischen Elements relativ zu einer Menge eines oder mehrerer anderer biologischer Elemente und/oder einem abgeleiteten Parameter eines oder mehrerer derselben, wobei das Verfahren vorzugsweise Ermitteln von zwei oder mehr Qualitätsparametern, wie mindestens drei Qualitätsparametern, wie mindestens vier Qualitätsparametern, umfasst.

4. Verfahren nach einem der Ansprüche 1-3, wobei das Verfahren Entnehmen der Partikel umfasst, wobei die Partikel vorzugsweise gemäß vorbestimmten Entnahmeanweisungen entnommen werden, wobei die vorbestimmten Entnahmeanweisungen vorzugsweise Anweisungen über Zeitfenster zum Entnehmen von Partikeln, Anweisungen über eine Entnahmezeit, Anweisungen über Standort und/oder Bewegung der Entnahmevorrichtung relativ zu Elementen der landwirtschaftlichen Gruppe, wie relativ zu Tieren der Tierherde oder relativ zu dem Futter der Mastanlage oder relativ zu Tierstreu der Tierstreuanlage, umfassen,
wobei das Verfahren vorzugsweise Entnehmen der Partikel unter Verwendung einer Entnahmevorrichtung, ausgewählt aus einer elektrostatischen Entnahmevorrichtung und/oder einer Filterentnahmevorrichtung, umfasst.

5. Verfahren nach einem der Ansprüche 1-4, wobei die Referenzdaten, wie der Referenzdatensatz/die Referenzdatensätze, mindestens eine Schwellenwertänderung der Menge des biologischen Elements als Funktion von Zeit nach dem Anfangsereignis, korreliert mit einem ausgewählten Niveau des Qualitätsparameters, darstellen,
wobei vorzugsweise die landwirtschaftliche Gruppe eine Tierherde ist und der Standort einer landwirtschaftlichen Gruppe ein Tierstandort ist, wobei das Anfangsereignis ausgewählt ist aus Schlüpfen, Geburt, Impfung, Medikamentengabe, Erfassung von mit Krankheit in Verbindung stehenden Erregern, Bewegung von Tieren, Änderung von Lichtverhältnissen, Futteränderung und/oder Ausbrüchen in benachbarten Herden, oder die landwirtschaftliche Gruppe eine Mastanlage ist und der Standort einer landwirtschaftlichen Gruppe ein Standort einer Mastanlage ist und/oder die landwirtschaftliche Gruppe eine Tierstreuanlage ist und der Standort einer landwirtschaftlichen Gruppe ein Standort einer Tierstreuanlage ist und wobei das Anfangsereignis ausgewählt ist aus einer Änderung der Temperatur, einer Änderung des Standorts, einer Änderung der Luftfeuchtigkeit, einer Änderung der Jahreszeit, einem Vermischen mit einer anderen Mastanlage und/oder einem Verstoß gegen ein Biosicherheitsprotokoll.

6. Verfahren nach einem der Ansprüche 1-5, wobei die landwirtschaftliche Gruppe eine Tierherde ist und der Standort einer landwirtschaftlichen Gruppe ein Tierstandort ist und wobei der Qualitätsparameter ein Qualitätsparameter der Tierherde ist und der Qualitätsparameter ein biologischer Parameter, ein Gesundheitsparameter, ein Wachstumsparameter und/oder ein Fleischqualitätsparameter, ein Produktionsparameter ist oder wobei die landwirtschaftliche Gruppe eine Mastanlage ist und der Standort einer landwirtschaftlichen Gruppe ein Standort einer Mastanlage ist, wobei der Qualitätsparameter ein Haltbarkeitsparameter, ein Frischeparameter, ein Schmackhaftigkeitsparameter, ein chemischer Parameter und/oder ein biologischer Parameter ist.

7. Qualitätssystem zum Ermitteln eines Qualitätsparameters einer landwirtschaftlichen Gruppe, das für die Verwendung in dem Verfahren nach einem der vorhergehenden Ansprüche geeignet ist, wobei das Qualitätssystem ein Computersystem (1) in Datenkommunikation mit einem Analysesystem (2) umfasst, das zur Analyse von Proben von Partikeln beschaffen ist, die aus Luft eines Standorts einer landwirtschaftlichen Gruppe, umfassend die landwirtschaftliche Gruppe, entnommen sind, wobei das Analysesystem zu Folgendem konfiguriert ist
- Empfangen einer Vielzahl von Proben, wobei jede Probe Partikel oder Partikelfragment(e) umfasst, die aus Luft an dem Standort einer landwirtschaftlichen Gruppe in einer Vielzahl von aufeinanderfolgenden, ausgewählten Zeitfenstern entnommen sind und jede Probe mit einem Zeitattribut korreliert ist, das einen Entnahmezeitpunkt darstellt;
- Durchführen mindestens einer quantitativen Ermittlung biologischer Elemente mit jeder der empfangenen Proben; und
- Übertragen von Teilsätzen von Daten, die jeweils Daten umfassen, die die mindestens eine quantitative Ermittlung biologischer Elemente einer Probe und das Zeitattribut der Probe darstellen, an das Computersystem;
wobei das Computersystem (1) zu Folgendem konfiguriert ist
- Empfangen der Teilsätze von Daten;
- Korrelieren der empfangenen Teilsätze von Daten mit mindestens einem Satz von Referenzdaten; und
- Ermitteln des Qualitätsparameters der landwirtschaftlichen Gruppe,
wobei der Satz von Referenzdaten eine Referenzmenge des biologischen Elements als Funktion von Zeit darstellt, die mit dem Qualitätsparameter korreliert ist, der mindestens einen Schwellenwert-Qualitätsparameter des mindestens einen biologischen Elements als Funktion von Zeit umfasst, und wobei die Ermittlung des Qualitätsparameters Ermitteln einer Menge des mindestens einen biologischen Elements als Funktion von Zeit umfasst und wobei das Computersystem (1) ferner zum Ermitteln des Qualitätsparameters relativ zu dem mindestens einen Schwellenwert-Qualitätsparameter konfiguriert ist,
wobei der Satz von Referenzdaten Ereignisdaten umfasst, die den Qualitätsparameter der landwirtschaftlichen Gruppe und den Standort einer landwirtschaftlichen Gruppe als Funktion von Zeit relativ zu einem Anfangsereignis darstellen,
wobei das Qualitätssystem optional mindestens einen Teil des Analysesystems umfasst.

8. Qualitätssystem nach Anspruch 7, wobei das Computersystem (1) und/oder ein Daten-Cloud-Speicher (4) in Datenkommunikation mit dem Computersystem (1) mindestens einen Satz von Referenzdaten speichert, wobei Sätze von Referenzdaten jeweils Referenzdaten umfassen, die eine Menge des biologischen Elements als Funktion von Zeit korreliert mit dem Qualitätsparameter darstellen, wobei der mindestens eine Satz von Referenzdaten vorzugsweise Referenzdaten umfasst, die mindestens einen Qualitätsparameter darstellen, der mindestens einer Art von landwirtschaftlicher Gruppe und/oder Art von Standort einer landwirtschaftlichen Gruppe zugeordnet ist und zum Anwenden als Referenzdaten zum Ermitteln des mindestens einen Qualitätsparameters für die jeweilige Art von landwirtschaftlicher Gruppe und/oder Art von Standort einer landwirtschaftlichen Gruppe geeignet ist.

9. Qualitätssystem nach Anspruch 7 oder Anspruch 8, wobei der Qualitätsparameter ein Mengenparameter oder ein abgeleiteter Mengenparameter ist, vorzugsweise ausgewählt aus einem Parameter der Menge des biologischen Elements, einem Parameter der Menge des biologischen Elements zu einem ausgewählten Zeitpunkt nach dem Anfangsereignis, einem Parameter der Menge des biologischen Elements relativ zu einem Schwellenwert, einem Parameter der Menge des biologischen Elements relativ zu einer Menge eines oder mehrerer anderer biologischer Elemente und/oder einem abgeleiteten Parameter eines oder mehrerer derselben.

10. Qualitätssystem nach einem der Ansprüche 7-9, wobei das Computersystem (1) und/oder der Daten-Cloud-Speicher (4) Daten speichert, die Anweisungen zum Entnehmen von Partikeln aus Luft darstellen; wobei die Daten, die Anweisungen zum Entnehmen von Partikeln aus Luft darstellen, vorzugsweise eine Datenbank mit Entnahmeanweisungen umfassen, die mit mindestens einer Art von landwirtschaftlicher Gruppe und/oder Art von Standort einer landwirtschaftlichen Gruppe korreliert sind; wobei die Entnahmeanweisungen vorzugsweise Anweisungen über Zeitfenster zum Entnehmen von Partikeln, Anweisungen über eine Entnahmezeit, Anweisungen über Standort und/oder Bewegung der Entnahmevorrichtung relativ zu Tieren der Tierherde oder relativ zu dem Futter der Mastanlage umfassen.

11. Qualitätssystem nach einem der Ansprüche 7-10, wobei das System eine Entnahmevorrichtung (3) umfasst, die zum Entnehmen mindestens teilweise in Luft schwebender Partikel geeignet ist, wobei die Entnahmevorrichtung eine elektrostatische Entnahmevorrichtung und/oder eine Filterentnahmevorrichtung ist und wobei mindestens ein Teil des Analysesystems (2) in die Entnahmevorrichtung (3) integriert ist, um bereitzustellen, dass die Analysevorrichtung zum Durchführen mindestens teilweise der mindestens einen quantitativen Ermittlung biologischer Elemente jeder der empfangenen Proben konfiguriert ist, wobei die Analysevorrichtung vorzugsweise zum Durchführen einer PCR-Amplifikation, Sequenzierung, Massenspektrometrie, Hochleistungsflüssigkeitschromatographie, Inkubation, mikrobiologischen Untersuchung und/oder optischen Identifizierung konfiguriert ist.

12. Qualitätssystem nach einem der Ansprüche 7-11, wobei das Computersystem (1) zum Empfangen der aufeinanderfolgenden Teilsätze der Daten in Echtzeit konfiguriert ist, wie sie von dem Analysesystem erzeugt werden, wobei das Computersystem (1) zum Korrelieren der empfangenen aufeinanderfolgenden Teilsätze der Daten in Echtzeit mit den Referenzdaten in Echtzeit, wie sie von dem Computersystem (1) empfangen werden, und vorzugsweise zum Durchführen der Ermittlung des Qualitätsparameters der landwirtschaftlichen Gruppe für jede der Korrelationen mit Referenzdaten konfiguriert ist.

13. Qualitätssystem nach einem der Ansprüche 7-12, wobei der Schwellenwert ein ausgewähltes Niveau des Qualitätsparameters ist, wobei das ausgewählte Niveau des Qualitätsparameters vorzugsweise ein Niveau ist, bei dem der Qualitätsparameter ein erhöhtes Risiko angibt, wie ein Risiko von geringer Produktion, geringem Wachstum, unerwünschter Infektion, Anstieg einer Sterblichkeitsrate, Verderb der Mastanlage, zoonotische Risiken und/oder jeden anderen Anstieg eines Risikos einer Qualitätsminderung.

14. Qualitätssystem nach einem der Ansprüche 7-13, wobei das Computersystem (1) zum Ermitteln einer Diagnoseangabe basierend auf dem ermittelten Qualitätsparameter konfiguriert ist, wenn sich der ermittelte Qualitätsparameter vorzugsweise außerhalb oder innerhalb eines Schwellenwertbereichs befindet und/oder wenn der Qualitätsparameter einen Schwellenwert überschreitet, wobei das Computersystem vorzugsweise zum Ermitteln einer Diagnose, einer Angabe einer Diagnose, einer Behandlung und/oder einer Angabe einer Behandlung zum Verbessern des Zustands der landwirtschaftlichen Gruppe basierend auf dem ermittelten Qualitätsparameter, wenn der Qualitätsparameter einen Schwellenwert überschreitet, konfiguriert ist.

15. Qualitätssystem nach einem der Ansprüche 7-14, wobei der mindestens eine Satz von Referenzdaten den Schwellenwert in Form mindestens einer Schwellenwertänderung der Menge des biologischen Elements als Funktion von Zeit ab dem Anfangsereignis umfasst und wobei die landwirtschaftliche Gruppe eine Tierherde ist und der Standort einer landwirtschaftlichen Gruppe ein Tierstandort ist, wobei das Anfangsereignis ausgewählt ist aus Schlüpfen, Geburt, Impfung, Medikamentengabe, Erfassung von mit Krankheit in Verbindung stehenden Erregern, Bewegung von Tieren, Änderung von Lichtverhältnissen, Futteränderung und/oder Ausbrüchen in benachbarten Herden.

16. Qualitätssystem nach einem der Ansprüche 7-14, wobei der mindestens eine Satz von Referenzdaten den Schwellenwert in Form mindestens einer Schwellenwertänderung der Menge des biologischen Elements als Funktion von Zeit ab dem Anfangsereignis umfasst und wobei die landwirtschaftliche Gruppe eine Mastanlage ist und der Standort einer landwirtschaftlichen Gruppe ein Standort einer Mastanlage ist oder die landwirtschaftliche Gruppe eine Tierstreuanlage ist und der Standort einer landwirtschaftlichen Gruppe ein Standort einer Tierstreuanlage ist und wobei das Anfangsereignis ausgewählt ist aus einer Änderung der Temperatur, einer Änderung des Standorts, einer Änderung der Luftfeuchtigkeit, einer Änderung der Jahreszeit, einem Vermischen mit einer anderen Mastanlage und/oder einem Verstoß gegen ein Biosicherheitsprotokoll.

## Revendications

1. Procédé de détermination d'un paramètre de qualité d'un groupe agricole situé au niveau d'un emplacement de groupe agricole et de préférence sélectionné parmi un troupeau d'animaux, un parc d'engraissement et/ou un parc de litière pour animaux, le procédé comprenant
- la fourniture d'une pluralité d'échantillons, chaque échantillon comprend des particules ou des fragments de particules collectés dans l'air dudit emplacement de groupe agricole à des intervalles de temps consécutifs et sélectionnés dans lequel chaque échantillon est corrélé à un attribut temporel représentant un temps de prélèvement ;
- la réalisation d'au moins une détermination d'élément biologique quantitative de chacun desdits échantillons pour obtenir des sous-ensembles de données comprenant pour chaque échantillon le résultat de ladite au moins une détermination et ledit attribut temporel pour ledit échantillon.
- la corrélation d'un ou de plusieurs desdits sous-ensembles de données avec des données de référence comprenant au moins un ensemble de données de référence ; et
- la détermination dudit paramètre de qualité du groupe agricole,
**caractérisé en ce que** ledit ensemble de données de référence représente une quantité de référence dudit élément biologique en fonction du temps corrélée audit paramètre de qualité comprenant au moins un paramètre de qualité seuil dudit au moins un élément biologique en fonction du temps et dans lequel la détermination dudit paramètre de qualité comprend la détermination d'une quantité dudit au moins un élément biologique en fonction du temps et dans lequel le procédé comprend également la détermination dudit paramètre de qualité par rapport audit au moins un paramètre de qualité seuil,
dans lequel l'ensemble de données de référence comprend des données d'événement représentant le paramètre de qualité du groupe agricole et l'emplacement de groupe agricole en fonction du temps par rapport à un événement initial.

2. Procédé selon la revendication 1, dans lequel ledit paramètre de qualité seuil comprend au moins un des éléments suivants
- une quantité seuil dudit au moins un élément biologique ;
- une dérive seuil de la quantité dudit au moins un élément biologique en fonction du temps et/ou
- un changement seuil de la quantité dudit élément biologique en fonction du temps, éventuellement corrélé à un niveau sélectionné dudit paramètre de qualité.

3. Procédé selon la revendication 1 ou la revendication 2, dans lequel le paramètre de qualité est un paramètre de quantité ou un paramètre de quantité dérivé, de préférence sélectionné parmi un paramètre de quantité dudit élément biologique, un paramètre de quantité dudit élément biologique à un moment sélectionné à partir de l'événement initial, un paramètre de quantité dudit élément biologique par rapport à un seuil, un paramètre de quantité dudit élément biologique par rapport à une quantité d'un ou de plusieurs autres éléments biologiques et/ou un paramètre dérivé d'un ou de plusieurs de ceux-ci, de préférence le procédé comprend la détermination de deux paramètres de qualité ou plus, par exemple au moins trois paramètres de qualité, par exemple au moins 4 paramètres de qualité.

4. Procédé selon l'une quelconque des revendications 1 à 3, dans lequel le procédé comprend la collecte desdites particules, de préférence lesdites particules étant collectées selon des instructions de collecte prédéterminées, lesdites instructions de collecte prédéterminées comprennent de préférence des instructions sur les intervalles de temps pour la collecte de particules, des instructions sur la durée de la collecte, des instructions sur l'emplacement et/ou le déplacement du collecteur par rapport aux éléments du groupe agricole, par exemple par rapport aux animaux du troupeau d'animaux ou par rapport à l'alimentation du parc d'engraissement ou par rapport à la litière pour animaux du parc de litière pour animaux,
de préférence, le procédé comprend la collecte desdites particules à l'aide d'un collecteur sélectionné parmi un collecteur électrostatique et/ou un collecteur à filtre.

5. Procédé selon l'une quelconque des revendications 1 à 4, dans lequel lesdites données de référence, par exemple ledit ou lesdits ensembles de données de référence, représentent au moins un changement seuil de la quantité dudit élément biologique en fonction du temps à partir de l'événement initial corrélé à un niveau sélectionné dudit paramètre de qualité,
de préférence, ledit groupe agricole est un troupeau d'animaux et ledit emplacement de groupe agricole est un emplacement d'élevage, dans lequel ledit événement initial est sélectionné parmi l'éclosion, la naissance, la vaccination, la médication, la détection d'agents pathogènes liés à une maladie, le déplacement d'animaux, un changement de réglage de l'éclairage, un changement d'alimentation et/ou des épidémies dans des troupeaux voisins ou ledit groupe agricole est un parc d'engraissement et ledit emplacement de groupe agricole est un emplacement de parc d'engraissement et/ou ledit groupe agricole est un parc de litière pour animaux et ledit emplacement de groupe agricole est un emplacement de parc de litière pour animaux et dans lequel ledit événement initial étant sélectionné parmi un changement de température, un changement d'emplacement, un changement d'humidité, un changement de saison, un mélange avec un autre parc d'engraissement et/ou une violation d'un protocole de biosécurité.

6. Procédé selon l'une quelconque des revendications 1 à 5, dans lequel le groupe agricole est un troupeau d'animaux et l'emplacement de groupe agricole est un emplacement d'élevage et dans lequel le paramètre de qualité est un paramètre de qualité du troupeau d'animaux et le paramètre de qualité est un paramètre biologique, un paramètre de santé, un paramètre de croissance et/ou un paramètre de qualité de la viande, un paramètre de production ou dans lequel le groupe agricole est un parc d'engraissement et ledit emplacement de groupe agricole est un emplacement de parc d'engraissement, dans lequel le paramètre de qualité est un paramètre de durabilité, un paramètre de croustillant, un paramètre d'appétibilité, un paramètre chimique et/ou un paramètre biologique.

7. Système de qualité pour déterminer un paramètre de qualité d'un groupe agricole approprié pour une utilisation dans le procédé selon l'une quelconque des revendications précédentes, le système de qualité comprenant un système informatique (1) en communication de données avec un système d'analyse (2) adapté à l'analyse d'échantillons de particules collectées dans l'air d'un emplacement de groupe agricole site comprenant le groupe agricole, dans lequel le système d'analyse est configuré
- pour la réception d'une pluralité d'échantillons, chaque échantillon comprend des particules ou des fragments de particules collectés dans l'air dudit emplacement de groupe agricole à une pluralité d'intervalles de temps consécutifs et sélectionnés et chaque échantillon étant corrélé à un attribut temporel représentant un temps de collecte ;
- pour réaliser au moins une détermination d'élément biologique quantitative pour chacun desdits échantillons reçus ; et
- pour la transmission de sous-ensembles de données, chacun comprenant des données représentant ladite moins une détermination d'élément biologique quantitative d'un échantillon et l'attribut temporel dudit échantillon audit système informatique ;
dans lequel le système informatique (1) est configuré
- pour recevoir lesdits sous-ensembles de données ;
- pour corréler lesdits sous-ensembles de données reçus avec au moins un ensemble de données de référence ; et
- pour déterminer ledit paramètre de qualité du groupe agricole, dans lequel ledit ensemble de données de référence représente une quantité de référence dudit élément biologique en fonction du temps corrélée audit paramètre de qualité comprenant au moins un paramètre de qualité seuil dudit au moins un élément biologique en fonction du temps et dans lequel la détermination dudit paramètre de qualité comprend la détermination d'une quantité dudit au moins un élément biologique en fonction du temps et dans lequel le système informatique (1) est également configuré pour déterminer ledit paramètre de qualité par rapport audit au moins un paramètre de qualité seuil,
dans lequel l'ensemble de données de référence comprend des données d'événement représentant le paramètre de qualité du groupe agricole et l'emplacement de groupe agricole en fonction du temps par rapport à un événement initial,
éventuellement le système qualité comprend au moins une partie dudit système d'analyse.

8. Système de qualité selon la revendication 7, dans lequel le système informatique (1) et/ou un stockage de données en nuage (4) en communication de données avec ledit système informatique (1) stockent ledit au moins un ensemble de données de référence, lesdits ensembles de données de référence comprennent chacun des données de référence représentant la quantité dudit élément biologique en fonction du temps corrélée audit paramètre de qualité, de préférence ledit au moins un ensemble de données de référence comprend des données de référence représentant au moins un paramètre de qualité associé à au moins un type de groupe agricole et/ou type d'emplacement de groupe agricole et approprié pour être appliqué pour des données de référence pour déterminer ledit au moins un paramètre de qualité pour ledit type de groupe agricole et/ou type d'emplacement de groupe agricole respectif.

9. Système de qualité selon la revendication 7 ou la revendication 8, dans lequel le paramètre de qualité est un paramètre de quantité ou un paramètre de quantité dérivé, de préférence sélectionné parmi un paramètre de quantité dudit élément biologique, un paramètre de quantité dudit élément biologique à un moment sélectionné à partir de l'événement initial, un paramètre de quantité dudit élément biologique par rapport à un seuil, un paramètre de quantité dudit élément biologique par rapport à une quantité d'un ou de plusieurs autres éléments biologiques et/ou un paramètre dérivé d'un ou de plusieurs de ceux-ci.

10. Système de qualité selon l'une quelconque des revendications 7 à 9, dans lequel le système informatique (1) et/ou le stockage de données en nuage (4) stockent des données représentant des instructions pour la collecte de particules dans l'air, de préférence les données représentant des instructions pour la collecte de particules dans l'air comprennent une base de données d'instructions de collecte corrélées à au moins un type de groupe agricole et/ou type d'emplacement de groupe agricole, de préférence les instructions de collecte comprennent des instructions sur les intervalles de temps de collecte des particules, des instructions sur l'heure de collecte, des instructions sur l'emplacement et/ou le mouvement du collecteur par rapport aux animaux du troupeau d'animaux ou par rapport à l'alimentation du parc d'engraissement.

11. Système de qualité selon l'une quelconque des revendications 7 à 10, dans lequel le système comprend un collecteur (3) adapté à la collecte au moins partiellement de particules en suspension dans l'air, dans lequel le collecteur est un collecteur électrostatique et/ou un collecteur à filtre et dans lequel au moins une partie du système d'analyse (2) est intégrée au collecteur (3) de manière à ce que l'analyseur soit configuré pour au moins réaliser partiellement l'au moins une détermination d'élément biologique quantitative de chacun desdits échantillons reçus, de préférence l'analyseur est configuré pour réaliser une amplification PCR, un séquençage, une spectrométrie de masse, une chromatographie liquide à haute pression, une incubation, un examen microbiologique et/ou une identification optique.

12. Système de qualité selon l'une quelconque des revendications 7 à 11, dans lequel le système informatique (1) est configuré pour recevoir en temps réel lesdits sous-ensembles consécutifs desdites données au fur et à mesure de leur génération par le système d'analyse, dans lequel le système informatique (1) est configuré pour corréler en temps réel lesdits sous-ensembles consécutifs desdites données avec lesdites données de référence au fur et à mesure de leur réception par le système informatique (1) et de préférence, pour chacune desdites corrélations avec les données de référence, réaliser ladite détermination dudit paramètre de qualité du groupe agricole.

13. Système de qualité de l'une quelconque des revendications 7 à 12, dans lequel ledit seuil est un niveau sélectionné dudit paramètre de qualité, de préférence ledit niveau sélectionné dudit paramètre de qualité est un niveau où le paramètre de qualité indique un risque accru, par exemple un risque de faible production, de faible croissance, d'infection indésirable, d'augmentation du taux de mortalité, de dégradation du parc d'engraissement, de risques zoonotiques et/ou toute autre augmentation du risque de diminution de la qualité.

14. Système de qualité selon l'une quelconque des revendications 7 à 13, dans lequel ledit système informatique (1) est configuré pour déterminer une indication de diagnostic usr la base du paramètre de qualité déterminé, lorsque ledit paramètre de qualité déterminé est de préférence en dehors ou à l'intérieur d'une plage seuil et/ou lorsque le paramètre de qualité dépasse un seuil,
de préférence dans lequel ledit système informatique, sur la base du paramètre de qualité déterminé, lorsque ledit paramètre de qualité déterminé dépasse un seuil, est configuré pour déterminer un diagnostic, une indication d'un diagnostic, un traitement et/ou une indication d'un traitement pour améliorer l'état du groupe agricole.

15. Système de qualité selon l'une quelconque des revendications 7 à 14, dans lequel ledit au moins un ensemble de données de référence comprend ledit seuil sous la forme d'au moins un changement seuil de la quantité dudit élément biologique en fonction du temps à partir de l'événement initial et dans lequel ledit groupe agricole est un troupeau d'animaux et ledit emplacement de groupe agricole est un emplacement d'élevage, ledit événement initial est sélectionné parmi l'éclosion, la naissance, la vaccination, la médication, la détection d'agents pathogènes liés à une maladie, le déplacement d'animaux, un changement de réglage de l'éclairage, un changement d'alimentation et/ou des épidémies dans des troupeaux voisins.

16. Système de qualité selon l'une quelconque des revendications 7 à 14, dans lequel ledit au moins un ensemble de données de référence comprend ledit seuil sous la forme d'au moins un changement seuil de la quantité dudit élément biologique en fonction du temps à partir de l'événement initial et dans lequel ledit groupe agricole est un parc d'engraissement et ledit emplacement de groupe agricole est un emplacement de parc d'engraissement ou ledit groupe agricole est un parc de litière pour animaux et ledit emplacement de groupe agricole est un emplacement de parc de litière pour animaux et dans lequel, ledit événement initial est sélectionné parmi un changement de température, un changement d'emplacement, un changement d'humidité, un changement de saison, un mélange avec un autre parc d'engraissement et/ou une violation d'un protocole de biosécurité.
